# EUROPEAN PATENT SPECIFICATION

(11) **EP 1 275 367 B1**
(45) Date of publication and mention of the grant of the patent: **29.11.2006**
(21) Application number: 01117774.8
(22) Date of filing: 01.08.2001
(51) Int. Cl.: A61K 8/49, A61Q 5/06

(54) **Hair coloring composition comprising pyrazoloazole compounds**
Haarfärbemittel enthaltend Pyrazoloazol-Farbstoffe
Colorations capillaires comprenant des colorants pyrazolazoliques

(30) Priority: 09.07.2001 EP 01115750
(43) Date of publication of application: 15.01.2003
(73) Proprietor: KAO CORPORATION, Chuo-ku Tokyo (JP)
(72) Inventor: Pratt, Dominic, Dr., 64295 Darmstadt (DE)
(74) Representative: HOFFMANN EITLE

(56) References cited:
- EP-A- 0 923 929
- EP-A- 1 240 892
- US-B1- 6 197 071

## Description

### Technical Field

The present invention relates to a hair coloring composition which comprises at least one specific direct dyestuff for coloring human hair, and also a method for coloring human hair by this composition.

### Background Art

Hair coloring compositions which comprise several anionic or cationic direct dyestuffs are well-known for a long time and, contrary to the permanent hair coloring compositions comprising oxidative dye intermediates, require no coupling reaction by oxidizing agents.

This type of direct coloring compositions is often applied to the hair as so-called toning shampoos together with surface-active components, or as lotions, emulsions or thickened solutions, i.e., gels.

This direct hair coloring composition should, however, be improved in its color durability and in particular intensity as well as the uniformity of the coloring and the color gloss.

Additionally, the above-described direct dyestuffs generally have the drawback that they are not compatible with alkaline peroxides, so the direct hair coloring compositions are generally applied to the hair without peroxide. Accordingly, the direct hair coloring composition has no bleaching effect on the hair.

### Disclosure of the Invention

An object of the present invention is to provide a hair coloring composition, on the basis of direct dyestuffs, which shows excellent dyeing characteristics and gives to the hair a durable and vivid coloring over a prolonged period of time and which can be applied to the hair in the form of a lotion, emulsion, solution, gel, suspension or also aerosol by adding propellants. Another object of the present invention is to provide a hair coloring composition which supplies also hair bleaching power coupled with the above-described performance, based on direct dyestuffs.

The solution to these objects is to provide such a composition comprising at least one specific direct dyestuff which has good hair coloring performance and compatibility with peroxides.

The present inventors have found that when the below-described pyrazoloazole direct dye is used as a hair dye, surprisingly, the resulting hair coloring composition can impart the hair with more vivid and durable color tone than general oxidative hair coloring compositions.

In addition, the present inventors have found that the below-described pyrazoloazole direct dyestuff has an excellent stability against alkaline peroxide, and that the hair coloring effect of the above-described composition comprising at least one of the below-described pyrazoloazole direct dyestuffs can be further improved when one applies this composition having a pH value of 7,5 to 12.5, together with at least one peroxide, because hair bleaching power derived from alkaline peroxide serves more vividness to the hair.

In one aspect of the present invention, there is thus provided a hair coloring composition comprising at least one direct dyestuff represented by the general formula (1) which has a pyrazoloazole structure, or a salt thereof; 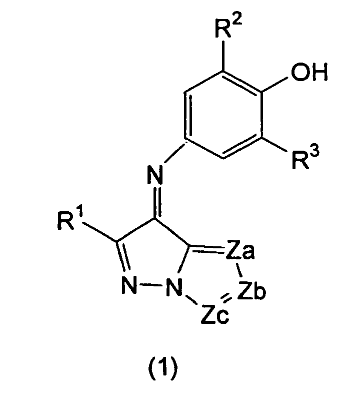 wherein, R¹ represents a hydrogen atom; a halogen atom; a C₁₋₅ alkyl group which may be optionally substituted by one or more than one halogen atom(s), hydroxy group(s), alkoxy group(s), aryloxy group(s), amino group(s), alkylamino group(s), acyl group(s), acylamino group(s); a C₁₋₄ alkoxy group; a C₁₋₄ alkylthio group; an arylthio group; a benzylthio group; an acyl group; an acylamino group; an acyloxy group; a carbamoyl group; a phenyl group which may be optionally substituted by one or more than one halogen atom(s), nitro group(s), sulfonyl group(s), C₁₋₄ alkyl group(s), C₁₋₄ alkoxy group(s), C₁₋₃ trifluoroalkyl group(s), amino group(s) or alkylamino group(s); an alkoxycarbonyl group; an aryloxycarbonyl group; a cyano group; a nitro group; a dialkylphosphinyl group; an alkylsulfinyl group; an arylsulfinyl group; a sulfamoyl group; a carboxyl group; a sulfo group; an aryloxy group; a C₁₋₄ alkylamino group; an ureido group; a sulfamoylamino group; a sulfonamido group; an alkoxycarbonylamino group; an aryloxycarbonylamino group; a heteroarylthio group; or a phosphonyl group,
each of Za, Zb and Zc independently represents a nitrogen atom or a carbon atom which has a group enumerated above for R¹; and only one of Zb and Zc is the carbon atom, each of R² and R³ independently represents a hydrogen atom or an electron withdrawing group.

In another aspect of the present invention, there is thus provided a method for coloring the hair by the use of the above-described hair coloring composition.

### Best Mode for Carrying out the Invention

As the pyrazoloazole direct dye of the present invention, those of the general formulas (2) or (3) which have pyrazolotriazole structure or a salt thereof; 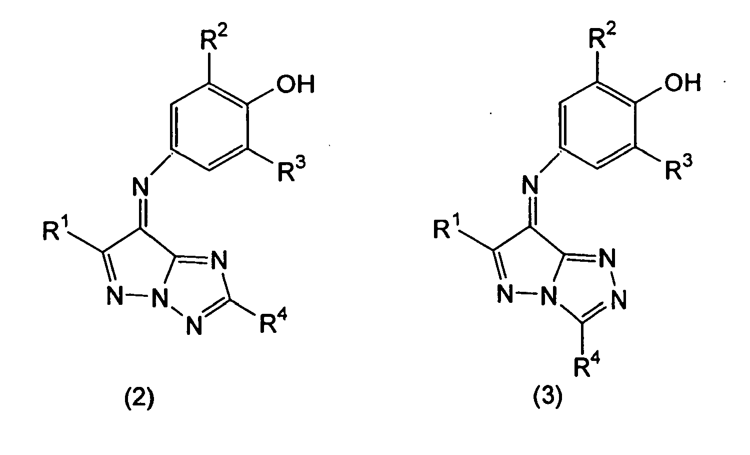 wherein, each of R¹ and R⁴ independently represents a hydrogen arom; a halogen atom; a C₁₋₅ alkyl group which may be optionally substituted by one or more than two halogen atom(s), hydroxy group(s), alkoxy group(s), aryloxy group(s), amino group(s), alkylamino group(s), acyl group(s), acylamino group(s); a C₁₋₄ alkoxy group; a C₁₋₄ alkylthio group; an arylthio group; benzylthio group; an acyl group; an acylamino group; an acyloxy group; a carbamoyl group; a phenyl group which may be optionally substituted by one or more than two halogen atom(s), nitro group(s), sulfonyl group(s), C₁₋₄ alkyl group(s), C₁₋₄ alkoxy group(s), C₁₋₃ trifluoroalkyl group(s), amino group(s) or alkylamino group(s); an alkoxycarbonyl group; an aryloxycarbonyl group; a cyano group; a nitro group; a dialkylphosphinyl group; an alkylsulfinyl group; an arylsulfinyl group; a sulfamoyl group; a carboxyl group; a sulfo group; an aryloxy group; a C₁₋₄ alkylamino group; an ureido group; a sulfamoylamino group; a sulfonamido group; an alkoxycarbonylamino group; an aryloxycarbonylamino group; a heteroarylthio group; or a phosphonyl group;
each of R² and R³ independently represents a hydrogen atom or an electron withdrawing group are in particular preferred.

The definitions for R¹ to R⁴ in the above-described formulas (1), (2) and (3) are explained as follows in more detail.

Examples of the halogen group represented by R¹ or R⁴ include a fluorine atom, chlorine atom, bromine atom and iodine atom. In particular, chlorine and bromine atoms are preferred.

Examples of the C₁₋₅ alkyl group represented by R¹ or R⁴ include methyl group, ethyl group, n-propyl group, 1-methylethyl group, n-butyl group, 1-methylpropyl group, t-butyl group and n-pentyl group.

Examples of the substituent which may optionally substitute on the above-described C₁₋₅ alkyl group includes chlorine atom, bromine atom, hydroxy group, methoxy group, ethoxy group, phenoxy group, amino group, methylamino group, dimethylamino group, 2-hydroxyethylamino group, bis(2-hydroxyethyl)amino group, acetyl group and acetylamino group. In particular, hydroxy group, methoxy group, ethoxy group, amino group, methylamino group, dimethylamino group, 2-hydroxyethylamino group and bis(2-hydroxyethyl)amino group are preferred in the light of the solubility of the dyestuff.

Examples of the C₁₋₄ alkoxy group represented by R¹ or R⁴ include methoxy group, ethoxy group, n-propyloxy group, 1-methylethoxy group, n-butoxy group, 1-methylpropyloxy group and t-butoxy group. In particular, methoxy group and ethoxy group are preferred.

Examples of the C₁₋₄ alkylthio group represented by R¹ or R⁴ include methylthio group and ethylthio group.

Examples of the arylthio group represented by R¹ or R⁴ include phenylthio group, 1-naphthylthio group, 2-naphthylthio group, 2-pyridylthio group, 3-pyridylthio group and 4-pyridylthio group.

Examples of the acyl group represented by R¹ or R⁴ include formyl group, acetyl group, hydroxyacetyl group, aminoacetyl group, and propionyl group.

Examples of the acylamino group represented by R¹ or R⁴ include formylamino group, acetylamino group and propionylamino group.

Examples of the acyloxy group represented by R¹ or R⁴ include acetyloxy group and propionyloxy group.

Examples of the substituent which may optionally substitute on the phenyl group represented by R¹ or R⁴ include fluorine atom, chlorine atom, bromine atom, iodine atom, nitro group, sulfonyl group, methyl group, ethyl group, methoxy group, ethoxy group, trifluoromethyl group, amino group, methylamino group, dimethylamino group, 2-hydroxyethylamino group and bis(2-hydroxyethyl)amino group. In particular, chlorine atom, bromine atom, amino group, methylamino group, dimethylamino group, 2-hydroxyethylamino group and bis(2-hydroxyethyl)amino group are preferred.

Examples of the alkoxycarbonyl group represented by R¹ or R⁴ include methoxycarbonyl group, ethoxycarbonyl group, propyloxycarbonyl group and 2-hydroxyethoxycarbonyl group.

Examples of the aryloxycarbonyl group represented by R¹ or R⁴ include phenoxycarbonyl group, 1-naphthyloxycarbonyl group, 2-naphthyloxycarbonyl group, 2-pydidyloxycarbonyl group, 3-pyridyloxycarbonyl group and 4-pyridyloxycarbonyl group.

Examples of the dialkylphosphinyl group represented by R¹ or R⁴ include dimethylphosphinyl group and diethylphosphinyl group.

Examples of the alkylsulfinyl group represented by R¹ or R⁴ include methylsulfinyl group and ethylsulfinyl group.

Examples of the arylsulfinyl group represented by R¹ or R⁴ include phenylsulfinyl group.

Examples of the aryloxy group represented by R¹ or R⁴ include phenoxy group, 1-naphthyloxy group, 2-naphthyloxy group, 2-pyridyloxy group, 3-pyridyloxy group and 4-pyridyloxy group.

Examples of the C₁₋₄ alkylamino group represented by R¹ or R⁴ include methylamino group, ethylamino group, n-propylamino group and n-butylamino group.

Examples of the alkoxycarbonylamino group represented by R¹ or R⁴ include methoxycarbonylamino group and ethoxycarbonylamino group.

Examples of the aryloxycarbonylamino group represented by R¹ or R⁴ include phenoxycarbonylamino group.

Examples of the heteroarylthio group represented by R¹ or R⁴ include 2-pyridylthio group, 2-imidazolothio group and 3-pyrazolothio group.

The group represented by R¹ or R⁴ may be a hydrogen atom. Furthermore, C₁₋₅ alkyl group which may be optionally substituted by one or more than two hydroxy group(s), alkoxy group(s), amino group(s) or alkylamino group(s); phenyl group which may be optionally substituted by one or more than two halogen atom(s), C₁₋₄ alkyl group(s), C₁₋₄ alkoxy group(s), amino group(s) or alkylamino group(s); are in particular preferred in the light of hair coloring intensity.

Particularly suitable examples of the electron withdrawing group represented by R² and R³ are fluorine atom, chlorine atom, bromine atom, iodine atom, carboxylic acid group, carboxylate group, alkoxycarbonyl group, a substituted or non-substituted aminocarbonyl group or an acyl group, wherein a fluorine atom, chlorine atom, bromine atom, iodine atom, carboxylic acid group and C₂₋₅ alkoxycarbonyl group are especially preferred in the light of hair coloring intensity.

Examples of the pyrazoloazole direct dye represented by formula (1) according to the present invention include the following structures.

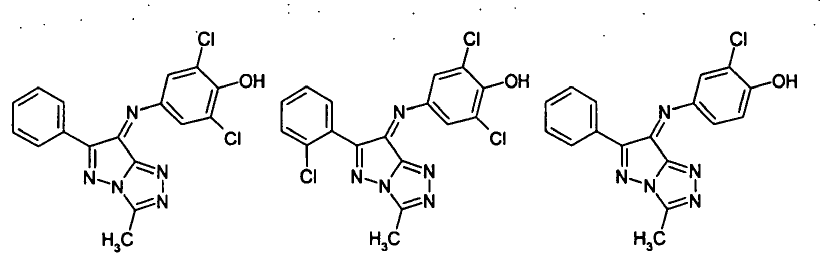

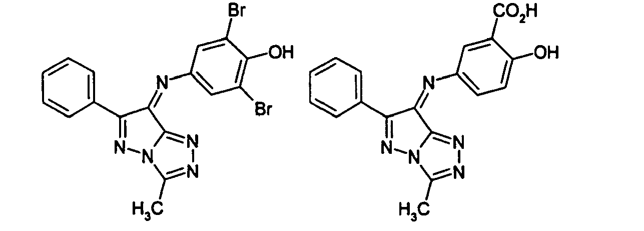

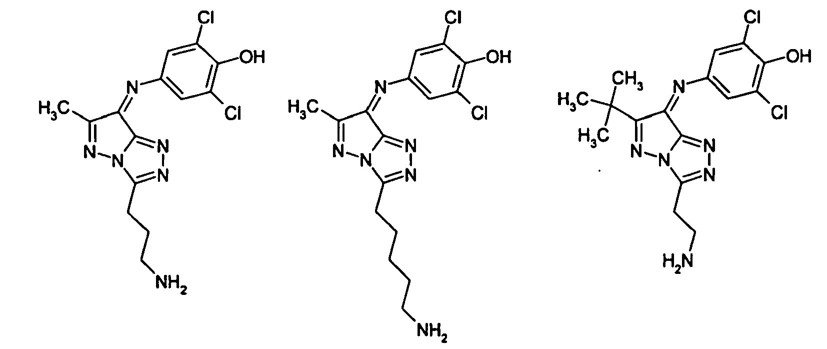

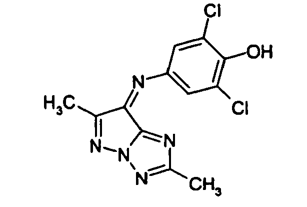

Each of the compounds of formula (1), (2) and (3) of the present invention may be a salt of an organic or inorganic acid, or an organic or inorganic alkali. Examples of the organic or inorganic acid include hydrochloric acid, sulfuric acid, phosphoric acid, acetic acid, propionic acid, lactic acid and citric acid. Examples of the organic or inorganic alkali include ammonium hydroxide, 2-ehanolammmonium hydroxide, sodium hydroxide and potassium hydroxide.

The production of the above-described pyrazoloazole direct dyestuffs is actually well-known and is achieved via oxidative coupling of the corresponding substituted pyrazoloazole with the corresponding substituted aminophenol derivatives.

The resulting colors are thereby in the range of intensive red to intensive magenta color, but they can be varied by further addition of colors.

The proportion of the above-described pyrazoloazole direct dyestuffs in the compositions according to the present invention is variable and depends on the structure and also on desired chroma; it is appropriate generally within about 0,001 to approximately 5, preferably 0,01 to 2,5, in particular 0,1 to 1 wt% based on the whole composition.

Beside the pyrazoloazole direct dye represented by the formula (1), additionally also further direct dyestuffs for hair can be along-used to create color nuances.

Concerning such direct dyestuffs, so-called "Arianor-dyestuffs" are preferred; referring to K.Schrader, "Grundlagen und Rezepturen der Kosmetika", 2^{nd} edition.(1989), p. 811. Particularly suitable basic (cationic) dyestuffs are:
- Basic Blue 6,: C.I.No. 51,175;
- Basic Blue 7,: C.I.No. 42,595;
- Basic Blue 9,: C.I.No. 52,015;
- Basic Blue 26,: C.I.No. 44,045;
- Basic Blue 41,: C.I.No. 11,154,
- Basic Blue 99,: C.I.No. 56,059;
- Basic Brown 4,: C.I.No. 21,010;
- Basic Brown 16,: C.I.No. 12,250;
- Basic Brown 17,: C.I.No. 12,251;
- Basic Brown 7,: C.I.No. 75,500;
- Basic Green 1,: C.I.No. 42,040;
- Basic Red 2,: C.I.No. 50,240;
- Basic Red 12,: C.I.No. 48,070;
- Basic Red 22,: C.I.No. 11,055;
- Basic Red 76,: C.I.No. 12,245;
- Basic Violet 1,: C.I.No. 42,535;
- Basic Violet 3,: C.I.No. 42,555;
- Basic Violet 10,: C.I.No. 45,170;
- Basic Violet 14,: C.I.No. 42,510;
- Basic Yellow 57,: C.I.No. 12,719;
as well as dyestuffs disclosed in patent EP 0618 464 B1.

Of course, also the use of direct dyestuffs from plants and/or anionic (acidic) direct dyestuffs for hair is possible.

The following dyestuffs can be used as suitable anionic dyestuffs:
- Acid Black 1,: C.I.No. 20,470;
- Acid Blue 1,: C.I.No. 42,045;
- Food Blue 5,: C.I.No. 42,051;
- Acid Blue 9,: C.I.No. 42,090;
- Acid Blue 74,: C.I.No. 73,015;
- Acid Red 18,: C.I.No. 16,255;
- Acid Red 27,: C.I.No. 16,185;
- Acid Red 87,: C.I.No. 45,380;
- Acid Red 92,: C.I.No. 45,410;
- Acid Orange 7,: C.I.No. 15,510;
- Acid Violet 43,: C.I.No. 60,730;
- Acid Yellow 1,: C.I.No. 10,316;
- Acid Yellow 23,: C.I.No. 19,140;
- Acid Yellow 3,: C.I.No. 47,005;
- Food Yellow No.8,: C.I.No. 14,270;
- D&C Brown No.1,: C.I.No. 20,170;
- D&C Green No.5,: C.I.No. 61,570;
- D&C Orange No.4,: C.I.No. 15,510;
- D&C Orange No.10,: C.I.No. 45,425:1;
- D&C Orange No.11,: C.I.No. 45,425;
- D&C Red No.21,: C.I.No. 45,380:2;
- D&C Red No.27,: C.I.No. 45,410:1;
- D&C Red No.33,: C.I.No. 17,200;
- D&C Yellow No.7,: C.I.No. 45,350:1;
- D&C Yellow No.8,: C.I.No. 45,350;
- FD&C Red No.4,: C.I.No. 14,700;
- FD&C Yellow No.6,: C.I.No. 15,985

Also the use of vegetable dyestuffs, which includes, for example, henna (red or black), alkanna root, laccaic acid (Stocklack), Indigo, logwood root powder, madder root and rhubarb powder, etc., alone or in combination with synthetic direct dyestuffs, is possible.

These direct dyestuffs can be usually likewise used in an amount of approximately 0,005 to approximately 5, preferably about 0,05 to approximately 2,5, in particular about 0,1 to approximately 1 wt% based on the whole composition, which is presented in the form of solution, dispersion, emulsion, gel or aerosol, for application.

The compositions according to the present invention can also comprise at least one oxidative dyestuff intermediate, i.e. a developer and/or a coupler substance.

Examples of the developer include, in particular, 1,4-diaminobenzene, 2,5-diaminotoluene, tetraaminopyrimidine, triaminohydroxypyrimidine, 1,2,4-triaminobenzene, 2-(2,5-diaminophenyl)ethanol, 2-(2-hydroxyethylamino)-5-aminotoluene, 1-amino-4-bis-(2-hydroxyethyl)aminobenzene or water-soluble salts thereof.

Examples of the coupler include resorcin, 2-methylresorcin, 4-chlororesorcin, 2-amino-4-chlorophenol, 4-(N-methylamino)phenol, 2-aminophenol, 3-aminophenol, 1-methyl-2-hydroxy-4-aminobenzene, 3-(N,N-dimethylamino)phenol, 4-amino-3-methylphenol, 5-amino-2-methylphenol, 6-amino-3-methylphenol, 3-amino-2-methylamino-6-methoxypyridine, 2-amino-3-hydroxypyridine, 4-aminodiphenylamine, 4,4'-diaminodiphenylamine, 2-dimethylamino-5-aminopyridine, 2,6-diaminopyridine, 1,3-diaminobenzene, 1-amino-3-(2-hydroxyethylamino)benzene, 1-amino-3-[bis(2-hydroxyethyl)amino]benzene, alpha-naphthol, 1,4-diamino-2-chlorobenzene, 4,6-dichlororesorcin, 1,3-diaminotoluene, 1-hydroxynaphthalene, 4-hydroxy-1,2-methylenedioxybenzene, 1,5-dihydroxynaphthalene, 1,7-dihydroxynaphthalene, 2,7-dihydroxynaphthalene, 2,4-diamino-3-chlorophenol and/or 1-methoxy-2-amino-4-(2-hydroxyethylamino)benzene. The above-described example enumerating could not limit the present invention on completeness.

Developers and couplers are preferably comprised in the molar ratio of 1:3 to 5:1, in particular about 1:1 to about 3:1, and their proportion incorporated in the hair coloring composition of the present invention can amount to about 0,05 to approximately 5 wt%, and it depends upon desired color.

The above-described pyrazoloazole direct dyestuff has an excellent stability against alkaline peroxide, so they are compatible with peroxide, which can provide hair bleaching ability at the same time as hair coloring. For this purpose, hair coloring composition of the present invention can comprise at least one peroxide with at least one above-described pyrazoloazole direct dyestuff, and the hair coloring composition can be preferably adjusted to pH value of 7,5 to 12.5 to provide a enough hair bleaching power.

As peroxides for the above-described hair coloring composition, all of diluted solutions, emulsions or gels of hydrogen peroxide can be used, but also further peroxides such as alkaline-earth peroxides, for example magnesium peroxide, urea peroxides or melaminperoxide, etc. can be used in appropriate stoichiometric quantities. Additionally, to increase the level of hair bleaching, in particular persulfates such as ammnonium persulfate, potassium persulfate and sodium persulfate can be incorporated in the composition having a pH value of approximately 7,5 to approximately 12,5, preferably about 8 to 11. The combined use of hydrogen peroxide and the above-described persulfates is preferable in the light of providing an exceptional bleaching power.

Preferable alkalization agents are ammnonia, monoethanolamine and alkali carbonates and -hydrogencarbonates such as sodium carbonate, potassium carbonate, sodium hydrogencarbonate and potassium hydrogencarbonate. However, also different alkalization agents such as di- and triethanolamine as well as further hydroxyalkanolamines can be used. As the hair-components which comprise persulfate, the components, which are mixed as powders with the aqueous hydrogen peroxide, e.g. 6 wt% of aqueous H₂O₂ solution, are particularly suitable referring, for example, the monography of K. Schrader, "Grundlagen und Rezepturen der Kosmetika", 2^{nd} edition (1989), pp. 815-823.

The above-described hair coloring composition, which comprises at least one oxidizing agent, can be applied in all well-known hair coloring media, in particular solutions, creams, emulsions, pastes, gels, (foam-)aerosols, etc.

The hair coloring composition of the present invention can also contain surface-active substances, and these should be used when it relates to a toning shampoo.

An anionic, nonionic, cationic, amphoteric or zwitter-inonic surfactant can be incorporated in the hair coloring composition of the present invention.

For other than toning shampoo, amphoteric or zwitter-ionic and cationic surfactants are preferably used in a quantity of between approximately 0,5 to about 5 wt% calculated on the whole composition. Suitable nonionic surfactants are selected from the class of the alkylpolyglucosides with the following general formula

R-O-(CH₂CH₂O)ₙ-Zₓ,

wherein R represents a C₈₋₂₀, preferably a C₁₀₋₁₄ alkyl group, Z represents a C₅₋₆ saccharide, n represents a number from 0 to 10, and x represents a number between 1 and 5, preferably 1,1 to 2,5. Further suitable nonionic surfactant for the composition of the present invention is C₁₀₋₂₂ fatty-alkyl ethoxylate.

Particularly suitable C₁₀₋₂₂ fatty-alcohol ethers are, under the trade names, the "Laureth", "Myristeth", "Oleth", "Ceteth", "Deceth", "Steareth" and "Ceteareth" after the CTFA nomenclature, which are added the number of the ethyleneoxides, e.g. Laureth-16:

The average number of the ethyleneoxides is appropriate thereby between about 2,5 to about 25, preferably about 10 to about 20.

Other additionally along-usable nonionic surfactants are e.g. sorbitan esters such as polyethyleneglycol-sorbitan stearyl acid ester, fatty acid polyglycol ester or also ester of fatty acid and mixed-polymerized polyglycol from ethylene oxide and propylene oxide, as they are on the market, for example, under the trade name "Pluronics®". Further additionally applicable surfactant is amine oxide.

Such amine oxides belong to the state of the art for a long time, for example to C₁₂₋₁₈ alkyldimethylamine oxide such as Lauryldimethylamine oxide, C₁₂₋₁₈ alkylamidopropylamine oxide or alkylamidoethylamine oxides, C₁₂₋₁₈ alkyldi(hydroxyethyl)amine oxide or alkyldi(hydroxypropyl)amine oxide or also amine oxides which have groups of ethylene oxides and/or propylene oxides in their alkyl chain. Suitable amine oxides are on the market, for example under the designations "Ammonyx®", "Aromox®" or "Genaminox®".

Further optional surfactant-constituents are fatty acid-mono and dialkanolamide, like cocofattyacid-monoethanolamide and myristic acid-monoisopropanolamide.

Suitable amphoteric or zwitter-ionic surfactants are, in particular, those of well-known betaines such as fattyacid-amidoalkylbetaine and sulfobetaine, for example Laurylhydroxysulfobetain; also long-chained alkylamino acids such as cocoaminoacetate, cocoaminopropionate, sodium cocoamphopropionate and sodium cocoamphoacetate are listed as suitable examples.

In particular betaines or sulfobetaines of the following structures; 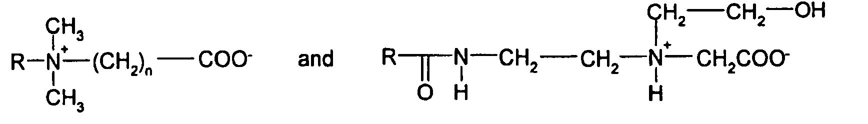 wherein R represents a C₈₋₁₈ alkyl group and n represents 1 to 3, 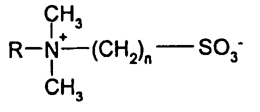 wherein R represents a C₈₋₁₈ alkyl group and n represents 1 to 3, 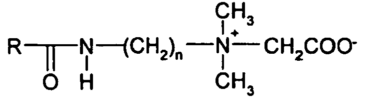 wherein R represents a C₈₋₁₈ alkyl group and n represents 1 to 3, can be used.

Preferable fattyacid-amidoalkylbetaines are, in particular, cocoamidopropylbetaine, cocoamphoacetate and cocoamphopropionate, and sodium salts thereof.

Preferable mixing ratio of cocoamidopropylbetaine and cocoamphoacetate is 3:1 to 1:3, and in particular, 2:1 to 1:1 in the weight ratio.

Suitable cationic surfactants are for example long-chained quaternized ammonium compounds, which can be used alone or in the combination together, like cetyltrimethylammonium chloride, dimethylstearylammonium chloride, trimethylcetylammonium bromide, stearyltrimethylammonium chloride, dimethylstearylbenzylammonium chloride, benzyltetradecyldimethylammonium chloride, dimethyl dihydrogenated tallow ammonium chloride, lauryldimethylbenzylammonium chloride, behenyltrimethylammonium chloride, lauryltrimethylammonium chloride, tris-(oligooxy-ethyl)alkylammonium phosphate, cetylpyridinium chloride, etc.

Quaternized ammonium salts disclosed in EP-A 472,107 are also well-suitable.

Further suitable long-chained ammonium compounds are esters of quartenized ammonium compounds represented by the general formula (4); 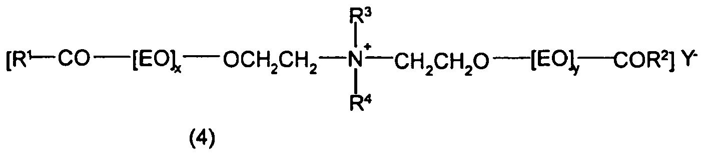 wherein R¹ and R² independently represent a C₈₋₂₂ alkyl group or alkenyl group which may optionally substututed by hydroxy group, R³ and R⁴ independently represent a C₁₋₃ alkyl group or a group - CH₂-CH₂-O-[EO]_{z}-H, x, y and z each independently represent 0 to 5 and Y⁻ represents an anion, and EO means ethyleneoxide.

It is particularly preferred that in the formula (4) R¹ and R² each represent an oleyl group or a C₁₂₋₁₈ alkyl group, R³ represents a methyl group and R⁴ represents a group - CH₂-CH₂-O-[EO]_{z}-H.

The anion Y⁻ is preferably a halide such as Cl⁻ or Br⁻, lower alkyl sulfate e.g. methyl sulfate and ethyl sulfate, or an alkyl phosphate, however, also general different anions can be assigned.

These compounds are well-known and on the market, for example, "Schercoquat®", "Dehyquart F30®" and "Tetranyl®" under the trade name. The application of these "Esterquats" for hair preservative agents is actually well-known and, for example, disclosed in WO-A 93/10748, WO-A 92/06799 and WO-A 94/16677.

Also suitable amides of quartenized ammonium compounds are represented by the formula (5): 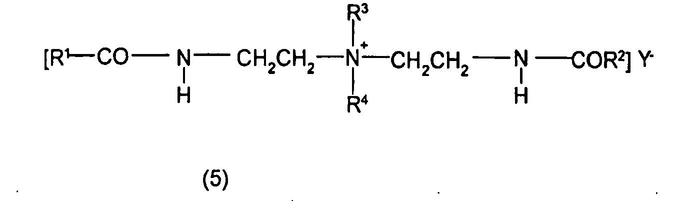 in that R¹ and R² represent a C₈₋₂₂ alkyl- or alkenyl group which may optionally substituted by a hydroxy group, R³ and R⁴ represent a C₁₋₃ alkyl group or a group - CH₂-CH₂-[EO]ₓ-H, as well as x represents 0 to 5 and Y⁻ represents an anion.

Generally known other surfactants can be also used, and the above-described surfactants are compatible together to be used.

A further desirable constituent for the hair coloring compositions of the present invention is a C₃₋₆ alkanediol or its ether, in particular, a mono-C₁₋₃ alkylether.

Preferential solvents for the composition of the present invention are 1,2- and 1,3-propanediol, 1-methoxy-2-propanol, 1-ethoxy-2-propanol, 1,3- and 1,4-butanediol, diethyleneglycol and its monomethyl and monoethyl ether as well as dipropyleneglycol and its monomethyl and monoethyl ether. The proportion of these diols are appropriate preferably between 0.5 and 30, more preferably about 1 to approximately 15, in particular about 5 to approximately 10 wt% on the whole composition. Beside the C₃₋₆ alkanediols or their ethers, also monoalcohols such as ethanol, 1-propanol and 2-propanol; polyalcohols such as glycerin and hexanetriol; ethylcarbitol, benzyl alcohol, benzyloxyethanol as well as propylene carbonate (4-methyl-1,3-dioxan-2-on), n-alkylpyrrolidone and urea can be additionally used. Further possible additional constituents are cationic, anionic, nonionic and amphoteric polymers, and they can be incorporated preferably in an amount of from approximately 0,1 to approximately 5, in particular, about 0,25 to 2,5 wt% of the whole coloring composition.

These compositions can comprise also further preservative agents such as oils and fats. Such are, for example, sun flower oil, almond oil, peach kernel oil, wheat germ oil, macadamia nut oil, Evening Primrose oil, jojoba oil, castor oil, or also olive or soybean oil, lanolin and its derivatives, likewise mineral oils such as paraffin oil and vaseline.

Synthetic oils and waxes, for example, silicone oils, polyethylene glycols, etc. can be used. Further suitable hydrophobic compounds, in particular, fatty acid esters such as Isopropyl myristate, - palmitate, - stearate and - isostearate, oleyl oleate, isocetyl stearate, hexyl laurate, dibutyl adipate, dioctyl adipate, myristyl myristate, oleyl erucate, polyethylene glycol and polyglyceryl fatty acid esters such as PEG-7-glycerylcocoate, cetyl palmitate, etc. can be also used.

If present in the form of an emulsion, the compositions of the present invention may comprise generally used emulsifying agents. The composition according to the present invention can comprise also long-chained fatty acids. As fatty acids, C₁₀₋₂₄, particularly C₁₂₋₂₂ fatty acids are preferable, and they can be incorporated in an amount of approximately 0,5 to 15 wt% in particular 1 to 10 wt%, calculated on the whole composition. Behenic acid and stearic acid are particularly suitable, however, other fatty acids for example myristic acid, palmitic acid, oleic acid or also mixtures of natural or synthetic fatty acids such as coco-fatty acid also can be incorporated.

The composition according to the present invention preferably has a viscosity of approximately 1,000 to approximately 60,000, in particular about 5,000 to 50,000, above all about 10,000 to 40,000 mPa.s at 20°C, measured by using the Brookfield rotary viscometer with a spindle of No.5 at 5rpm.

The pH value is adjusted preferably to a basic range of between approximately 7,5 and 12,5 for example between 7.8 and 12, in particular between about 8 and 11. When it relates to toning shampoos, it can generally comprise, beside the above-enumerated surface-active substances, anionic surfactant.

Suitable ones of anion-active surfactant are comprised in the composition in an amount of at least 5 to approximately 50 wt%, preferably 10 to 25 wt%.

Examples of anion-active surfactant include such of sulfate-, sulfonate-, carboxylate- and alkyl phosphate-type, above all those which are usually used in shampoos. For example, the well-known C₁₀₋₁₈ alkyl sulfates and in particular the appropriate ether sulfates, for example C₁₂₋₁₄ alkylether sulfate, laurylether sulfate, in particular with 1 to 4 ethyleneoxide groups in the molecule can be listed. Furthermore, monoglyceride(ether)sulfate, fatty acid amide sulfates which are produced by ethoxylation and following sulfate introduction to the corresponding fatty acid alkanolamide, and their alkali salts as well as salts of long-chained mono and dialkyl phosphates, which represent mild detergent and which can be applied on the skin, also can be used.

Further suitable anionic surfactant is alpha-olefinsulfonate or its salts, and in particular alkali salts of sulfosuccinic acid half-ester, for example disodium salt of the monooctylsulfo succinate and alkali salts of long-chained monoalkylethoxysulfo succinate.

Suitable surfactant of the carboxylate-type is alkylpolyethercarbonic acid or their salts of the following formula;

R-(C₂H₄O)ₙ-O-CH₂COOX

wherein R represents a C₈₋₂₀ alkyl group, preferably a C₁₂₋₁₄ alkyl group, n represents a number of 1 to 20, preferably 2 to 17, and X represents a hydrogen atom or a cation of sodium, potassium, magnesium or ammonium, and which can be optionally hydroxyalkyl-substituted, as well as alkamidopolyethercarboxylic acid of the following general formula; 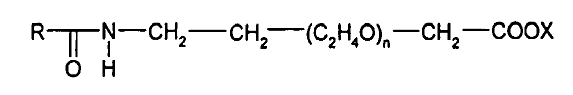 wherein R and X represent the same meanings as described above, and n represents in particular a number from 1 to 10, preferably 2,5 to 5.

Such products are well-known and on the market for a long time, for example "AKYPO®" and "AKYPO-SOFT®" under the trade name.

Also C₈₋₂₀ acyl isethionate and likewise sulfofatty acid and their ester can be used, however, in the mixture with other surfactants.

Also mixtures of several anionic surfactants, for example mixtures of an alpha-olefin sulfate and a sulfo succinate, preferably in the relation of 1:3 to 3:1, or an ether sulfate and a polyethercarboxylic acid or an alkylamidoethercarboxylic acid can be used.

An outline of the anion-active surfactants which are used as liquid body cleaning agents is disclosed in the monography of K.Schrader, "Grundlagen und Rezepturen der Kosmetika", 2^{nd} edition (1989, Huethig Buchverlag), pp. 683 to 691.

Concerning the concentration of anionic surfactant, the preferable amount thereof in toning shampoos of the present invention is between approximately 5 and about 30 wt%, in particular between approximately 7,5 to approximately 25 wt%, particularly preferentially between approximately 10 to approximately 20 wt%, calculated on the whole composition.

A preferable execution form of the toning shampoos according to the present invention contains preferably a mixture of at least one anionic surfactant above-mentioned and a C₈₋₂₂ acylaminocarbonic acid or their water-soluble salts, preferably in a quantity from 0.5 to 10, in particular 1 to 7,5 wt%, calculated on the whole composition.

Particularly preferable one is the N-lauroylglutamate, in particular the sodium salt thereof. Further suitable N-acylaminocarbonic acids are, for example, N-lauroylsarcosinate, N-C₁₂₋₁₈-acyl asparaginic acid, N-myristoylsarcosinate, N-oleoylsarcosinate, N-lauroylmethylalanine, N-lauroyllysine and N-lauroylaminopropylglycine, preferably in the form of their water-soluble alkali salt or ammonium salt, in particular sodium salts.

The composition of the present invention can further comprise well-known raw materials for cosmetics, such as thickeners, perfumes, stabilizers, solubilizing-aid agents, natural and/or synthetic polymers, chelating agents, etc. The composition of these substances is basically well-known and for example described in the monography by K. Schrader, I.c., pp. 796-798.

The hair coloring composition of the present invention can be prepared in a conventional manner into a one-part composition, a two-part composition having a first-part component containing an alkalization agent and a second-part component containing a oxidizing agent, or a three-part composition having, in addition to these two components, a powdery oxidizing agent such as persulfate. The direct pyrazoloazole dye (1) can be incorporated in either one or both of these components of the two-part or three-part composition. The one-part type is applied to the hair directly, while the two- or three-part type is applied to the hair after mixing these parts upon hair coloring.

The induction period of this hair coloring composition is preferably approximately 10 to 30 minutes; it can be reduced by thermal effect. Afterwards the hair is washed or rinsed and dried.

The following examples serve to illustrate the present invention.

The compounds obtained according to the synthetic examples I to V and the examples using the dyes of I, II, III, IV and V are according to the present invention. On the other hand, the compounds of the examples obtained in accordance with the synthetic examples VI and VII are not according to the present invention, these serve for comparison purposes only.

### -Examples-

### Production example I

6-Methyl-3-phenyl-1H-pyrazolo(5,1-c)(1,2,4)triazole (0.5g) was dissolved in alkaline water at pH 12 (50mls.) To this was added 4-amino-2,6-dichlorophenol (0.5g) with stirring. To this mixture was added potassium persulphate (0.2g), upon which, the solution turned dark purple. The mixture was left with stirring for 2hrs, before collecting the resulting precipitate via filtration. The dark brown precipitate was dried and then taken up in acetone. The acetone solution was filtered to remove impurities and then evaporated to leave pyrazolotriazole (I) as a dark brown crystalline powder.

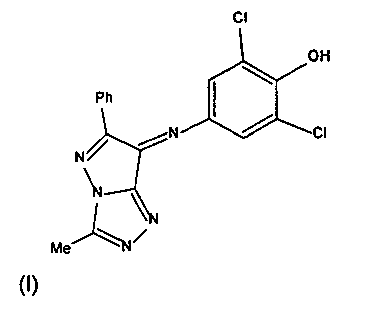

### Production examples II - V

6-Methyl-3-(2-chlorophenyl)-1H-pyrazolo(5,1-c)(1,2,4)triazole (0.5g) was dissolved in alkaline water at pH 12 (50mls.) To this was added 4-amino-2,6-dichlorophenol (0.5g) with stirring. To this mixture was added potassium persulphate (0.2g), upon which, the solution turned dark purple. The mixture was left with stirring for 2hrs, before collecting the resulting precipitate via filtration. The dark brown precipitate was dried and then taken up in acetone. The acetone solution was filtered to remove impurities and then evaporated to leave pyrazolotriazole (II) as a dark brown crystalline powder.

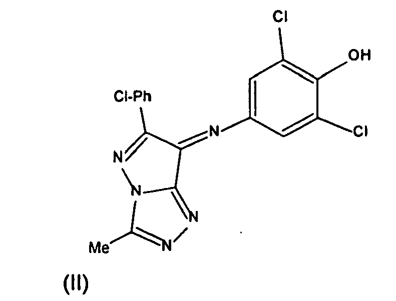

Compounds (III), (IV) and (V) were produced with similar method of production examples (I) and (II).

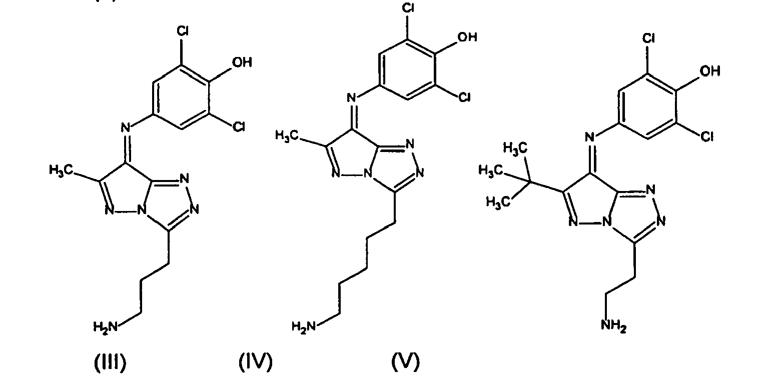

### Production example VI (not according to the invention)

6-Methyl-3-(2-chlorophenyl)-1H-pyrazolo(5,1-c)(1,2,4)triazole (0.5g) was dissolved in alkaline water at pH 12 (50mls.) To this was added 2,5diaminotoluene (0.5g) with stirring. To this mixture was added potassium persulphate (0.2g), upon which, the solution turned dark purple. The mixture was left with stirring for 2hrs, before collecting the resulting precipitate via filtration. The dark brown precipitate was dried and then taken up in acetone. The acetone solution was filtered to remove impurities and then evaporated to leave pyrazolotriazole (VI) as a dark brown crystalline powder.

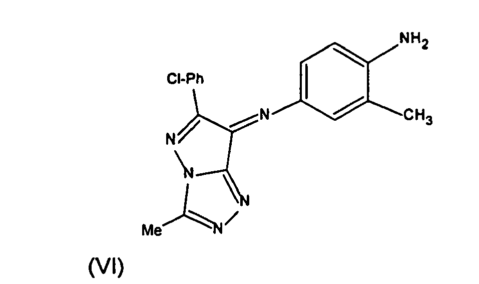

### Production example VII (not according to the invention)

6-Methyl-3-(2-chlorophenyl)-1H-pyrazolo(5,1-c)(1,2,4)triazole (0.5g) was dissolved in alkaline water at pH 12 (50mls.) To this was added N,N-diethyl-p-phenylenediamine (0.5g) with stirring. To this mixture was added potassium persulphate (0.2g), upon which, the solution turned dark purple. The mixture was left with stirring for 2hrs, before collecting the resulting precipitate via filtration. The dark brown precipitate was dried and then taken up in acetone. The acetone solution was filtered to remove impurities and then evaporated to leave pyrazolotriazole (VII) as a dark brown crystalline powder.

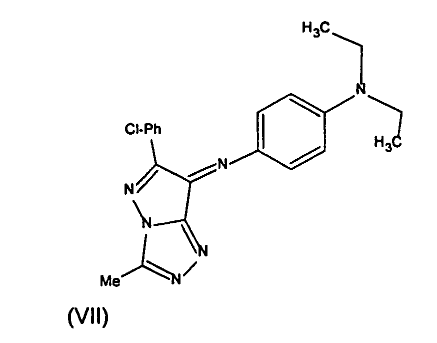

### Examples 1 and 2 -Dyeing

### Example 1

Pyrazolotriazole derived direct dye (I) (0.01g) was dissolved in the minimum amount of ethanol (0.5g). The dark magenta solution was transferred into a second aqueous solution containing benzyl alcohol (0.5g) and Water (9g) and adjusted to pH 10 with ammonia. An undamaged white goat hair tress was submerged in the solution for 20mins at 30°C. After this time the tress was shampoo rinsed and then dried.

### Example 2

Pyrazolotriazole derived direct dye (II) (0.01g) was dissolved in the minimum amount of ethanol (0.5g). The dark magenta solution was transferred into a second aqueous solution containing benzyl alcohol (0.5g) and Water (9g) and adjusted to pH 10 with ammonia. An undamaged white goat hair tress was submerged in the solution for 20mins at 30°C. After this time the tress was shampoo rinsed and then dried.

### Comparative example 1 (not according to the invention)

Pyrazolotriazole derived direct dye (VI) (0.005g) was dissolved in the minimum amount of ethanol (0.5g). The dark magenta solution was transferred into a second aqueous solution containing benzyl alcohol (0.5g) and Water (9g) and adjusted to pH 10 with ammonia. An undamaged white goat hair tress was submerged in the solution for 20mins at 30°C. After this time the tress was shampoo rinsed and then dried.

### Comparative example 2 (not according to the invention)

Pyrazolotriazole derived direct dye (VII) (0.005g) was dissolved in the minimum amount of ethanol (0.5g). The dark magenta solution was transferred into a second aqueous solution containing benzyl alcohol (0.5g) and Water (9g) and adjusted to pH 10 with ammonia. An undamaged white goat hair tress was submerged in the solution for 20mins at 30°C. After this time the tress was shampoo rinsed and then dried.

Lab values of resulting colored tress were measured by Minolta color-measuring instrument and the value of delta E, which is a measure for the chroma as well known, was calculated in usual way for each example (this will apply equally to every examples hereinafter). Table 1 shows the results.

**Table 1**

| **Dye** | **L** | **a** | **b** | **Delta E** | **Coloring result** |
|---|---|---|---|---|---|
| Before | 80 | 1 | 14 | - | White goat hair |
| Dye(I) of example 1 | 47 | 46 | -21 | 66 | Bright vivid magenta |
| Dye(II) of example 2 | 46 | 49 | -22 | 69 | Bright vivid magenta |
| Dye(VI) of comparative example 1 | 70 | 13 | 5 | 18 | Dull light pink |
| Dye(VII) of comparative example 2 | 65 | 12 | -2 | 24 | Dull light pink |

Each composition of example 1 and 2 imparted a bright vivid magenta color, while composition of comparative example 1 and 2 imparted only a dull color with very week pink tones.

These comparing examples show surprising superiority of the direct dyes according to the present invention in contrast to the structurally similar direct dyes of comparative examples 1 and 2.

### Examples 3 to 8 -Dyeing

A base of the following composition was prepared.

**Table 2**

| **Example** | **3** | **4** | **5** | **6** | **7** | **8** |
|---|---|---|---|---|---|---|
| Dye | (I) | (I) | (III) | (III) | (IV) | (V) |
| | 0.005g | 0.02g | 0.005g | 0.02g | 0.005g | 0.005g |
| Ethanol | 0.5g | 0.5g | 0.5g | 0.5g | 0.5g | 0.5g |
| Ammonia (35%) | 0.5g | 0.5g | 0.5g | 0.5g | 0.5g | 0.5g |
| Benzyl alcohol | 0.5g | 0.5g | 0.5g | 0.5g | 0.5g | 0.5g |
| Wate up to | 10g | 10g | 10g | 10g | 10g | 10g |

Each composition was applied to white goat tresses for 15mins at 50°C. After this time the tress was shampoo rinsed and then dried.

Table 3 shows the results.

**Table 3**

| **Example** | **L** | **a** | **b** | **Coloring result** |
|---|---|---|---|---|
| 3 | 47 | 43 | -22 | Bright magenta |
| 4 | 29 | 50 | -17 | Bright, intense magenta |
| 5 | 53 | 52 | -6 | Bright red-pink |
| 6 | 40 | 61 | -8 | Bright intense red-pink |
| 7 | 47 | 53 | -10 | Bright red-pink |
| 8 | 55 | 30 | -2 | Red-magenta |

Each composition imparted a bright color with red-pink to magenta tones.

### Examples 9 and 10 -Dyeing with hydrogen peroxide

### Example 9

Pyrazolotriazole derived direct dye (I) (0.012g) was dissolved in the minimum amount of ethanol (0.5g). The dark magenta solution was transferred into a second aqueous solution containing benzyl alcohol (0.5g) and Water (9g) and adjusted to pH 10 with ammonia. To this was added 50% hydrogen peroxide (0.65g) and the pH readjusted to 10 with ammonia. An undamaged white goat hair tress was submerged in the solution for 20mins at 30C. After this time the tress was shampoo rinsed and then dried.

### Example 10

Pyrazolotriazole derived direct dye (II) (0.01g) was dissolved in the minimum amount of ethanol (0.5g). The dark magenta solution was transferred into a second aqueous solution containing benzyl alcohol (0.5g) and Water (9g) and adjusted to pH 10 with ammonia. To this was added 50% hydrogen peroxide (0.65g) and the pH readjusted to 10 with ammonia. An undamaged white goat hair tress was submerged in the solution for 20mins at 30C. After this time the tress was shampoo rinsed and then dried.

Table 4 shows the results.

**Table 4**

| **Dye** | **L** | **a** | **b** | **Delta E** | **Coloring result** |
|---|---|---|---|---|---|
| Before | 80 | 1 | 14 | / | White goat hair |
| Dye(I) of example 9 | 40 | 48 | -26 | 73.5 | Bright vivid magenta |
| Dye(II) of example 10 | 48 | 50 | -27 | 71.5 | Bright vivid magenta |

Each composition of example 9 and 10 imparted a bright vivid magenta color.

### Examples 11 -Compatibility with oxidative technology

A base of the following composition was prepared.

**Table 5**

| **Example** | **11** | **Comparative example 3** |
|---|---|---|
| pyrazolotriazole dyestuff (III) | 0.02 | 0 |
| p-phenylenediamine | 0.05 | 0.05 |
| resorcinol | 0.05 | 0.05 |
| o-aminophenol | 0.4 | 0.4 |
| p-aminophenol | 0.4 | 0.4 |
| Benzyl alcohol | 5.0 | 5.0 |
| Ethanol | 5.0 | 5.0 |
| Peroxide (50%) | 6.0 | 6.0 |
| Ammonium Hydroxide (30%) | to pH 10 | to pH 10 |
| Water | up to 100 | 100 |

Both compositions applied to white goat tresses for 15mins at 50°C. After this time the tress was shampoo rinsed and then dried.

Table 6 shows the results.

**Table 6**

| | **Coloring result** | | |
|---|---|---|---|
| Example11 | L= 32 | a= **13** | b= 8 |
| Comparative example 3 | L= 31 | a= **7** | b= 13 |

Composition of example 11 imparted a red auburn colour, while composition of comparative example 3 imparted a light brown with no red tones.

### Examples 12 and 13 -Compatibility with persulfate

A base of the following composition was prepared.

**Table 7**

| Example | **12** | **13** |
|---|---|---|
| Dyestuff (III) | 0.1 | 0.0 |
| Dyestuff (I) | 0.0 | 0.1 |
| Benzyl alcohol | 10 | 10 |
| Ethanol | 5.0 | 5.0 |
| Ammonium persulphate | 0.5 | 0.5 |
| Potassium persulphate | 0.5 | 0.5 |
| Peroxide (50%) | 6.0 | 6.0 |
| Ammonium hydroxide (35%) | to pH 10 | to pH 10 |
| Water | up to 100 | up to 100 |

Composition 12 and 13 was applied to black Chinese hair tresses and left for 30mins at 50°C. After this time the tress was shampoo rinsed and then dried.

Table 8 shows the results.

**Table 8 Coloring result**

| | L | a | b |
|---|---|---|---|
| Before | 19 | **1.5** | 1.9 |
| Example 12 | 29 | **27** | 10 |
| Example 13 | 25 | **33** | 0.2 |

Composition of example 12 imparted a bright red coloration whilst composition of example 13 imparted a vivid magenta colour. Both compositions delivered significant bleaching with intense red coloration.

Surprisingly the direct dyes in accordance with the present invention have better properties when compared with structurally similar compounds.

The hair coloring compositions according to the present invention also provide the longer lasting brilliant colors based on characteristics of pyrazoloazole direct dyes against hair washing and environmental influences such as exposure to the sun, sweat, rain, etc. when compared with conventional direct dyestuffs.

## Claims

1. A hair coloring composition comprising, as a direct dyestuff, a pyrazoloazole direct dyestuff represented by the following general formula (1) or a salt thereof: 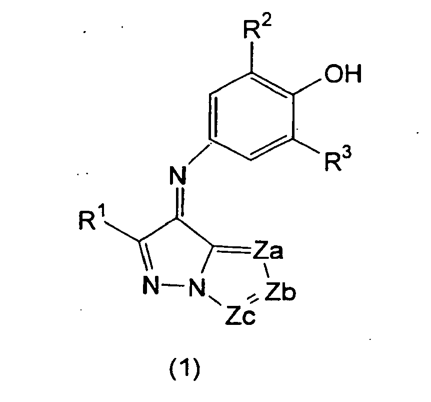 wherein, R¹ represents a hydrogen arom; a halogen atom; a C₁₋₅ alkyl group which may be optionally substituted by one or more than one halogen atom(s), hydroxy group(s), alkoxy group(s), aryloxy group(s), amino group(s), alkylamino group(s), acyl group(s), acylamino group(s); a C₁₋₄ alkoxy group; a C₁₋₄ alkylthio group; an arylthio group; benzylthio group; an acyl group; an acylamino group; an acyloxy group; a carbamoyl group; a phenyl group which may be optionally substituted by one or more than one halogen atom(s), nitro group(s), sulfonyl group(s), C₁₋₄ alkyl group(s), C₁₋₄ alkoxy group(s), C₁₋₃ trifluoroalkyl group(s), amino group(s) or alkylamino group(s); an alkoxycarbonyl group; an aryloxycarbonyl group; a cyano group; a nitro group; a dialkylphosphinyl group; an alkylsulfinyl group; an arylsulfinyl group; a sulfamoyl group; a carboxyl group; a sulfo group; an aryloxy group; a C₁₋₄ alkylamino group; an ureido group; a sulfamoylamino group; a sulfonamido group; an alkoxycarbonylamino group; an aryloxycarbonylamino group; a heteroarylthio group; or a phosphonyl group. Each of Za, Zb and Zc indepentently represents a nitrogen atom or a carbon atom which has a group renumerated above for R¹; and only one of Zb and Zc is the carbon atom, each of R² and R³ independently represents a hydrogen atom or an electron withdrawing group.

2. A hair coloring composition according to claim 1, wherein the pyrazoloazole direct dyestuff or the salt thereof is represented by the following general formula (2) or (3) or a salt thereof: 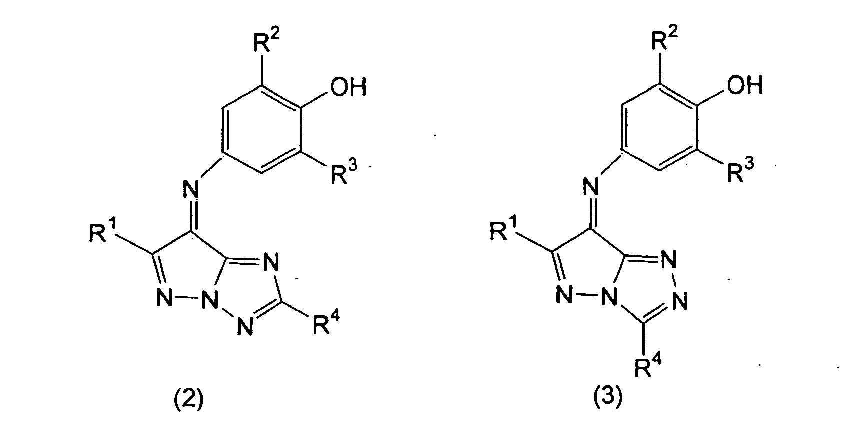 wherein, R¹ and R⁴ independently represents a hydrogen arom; a halogen atom; a C₁₋₅ alkyl group which may be optionally substituted by one or more than one halogen atom(s), hydroxy group(s), alkoxy group(s), aryloxy group(s), amino group(s), alkylamino group(s), acyl group(s), acylamino group(s); a C₁₋₄ alkoxy group; a C₁₋₄ alkylthio group; an arylthio group; benzylthio group; an acyl group; an acylamino group; an acyloxy group; a carbamoyl group; a phenyl group which may be optionally substituted by one or more than one halogen atom(s), nitro group(s), sulfonyl group(s), C₁₋₄ alkyl group(s), C₁₋₄ alkoxy group(s), C₁₋₃ trifluoroalkyl group(s), amino group(s) or alkylamino group(s); an alkoxycarbonyl group; an aryloxycarbonyl group; a cyano group; a nitro group; a dialkylphosphinyl group; an alkylsulfinyl group; an arylsulfinyl group; a sulfamoyl group; a carboxyl group; a sulfo group; an aryloxy group; a C₁₋₄ alkylamino group; an ureido group; a sulfamoylamino group; a sulfonamido group; an alkoxycarbonylamino group; an aryloxycarbonylamino group; a heteroarylthio group; or a phosphonyl group,
each of R² and R³ independently represents a hydrogen atom or an electron withdrawing group.

3. A hair coloring composition according to claim 2, wherein in the general formula (2) or (3) R¹ and R⁴ independently represents a hydrogen atom; a C₁₋₅ alkyl group which may be optionally substituted by one or more than one hydroxy group(s), alkoxy group(s), amino group(s) or alkylamino group(s); or a phenyl group which may be optionally substituted by one or more than two halogen atom(s), C₁₋₄ alkyl group(s), C₁₋₄ alkoxy group(s), amino group(s) or alkylamino group(s); R² and R³ independently represents a hydrogen atom, a fluorine atom, a chlorine atom, a bromine atom, an iodine atom, a carboxylic acid group or a C₁₋₄ alkoxycarbonyl group.

4. A hair coloring composition according to any one of the claims 1 to 3 wherein the direct dyestuff is represented by the following formulas:

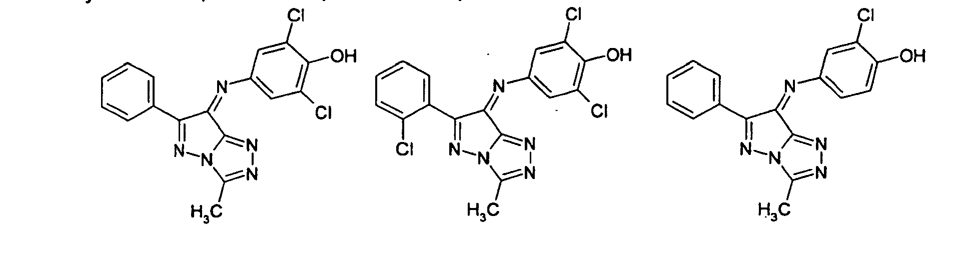

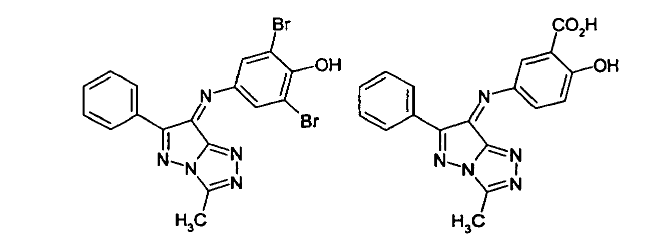

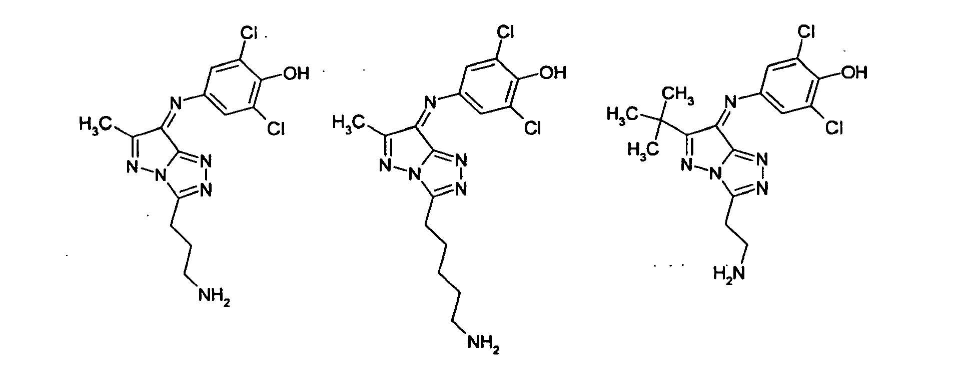

5. A hair coloring composition according to any one of the claims 1 to 4, which comprises 0,001 to 5 wt% of at least one pyrazoloazole direct dyestuff based on the whole composition.

6. A hair coloring composition according to any one of the claims 1 to 5, which further comprises at least one oxidative dye intermediate.

7. A hair coloring composition according to any one of the claims 1 to 6, which further comprises at least one peroxide and has a pH value of approximately 7,5 to approximately 12,5.

8. A hair coloring composition according to claim 7, which comprises hydrogen peroxide as the peroxide.

9. A hair coloring composition according to claim 7, which comprises at least one of ammonium persulfate, sodium persulfate and potassium persulfate as peroxide.

10. A hair coloring composition according to claim 7, which comprises both of hydrogen peroxide and at least one of ammonium persulfate, sodium persulfate and potassium persulfate as peroxide.

11. A method for coloring the hair with a hair coloring composition as claimed in any one of claims 1 to 10.

## Patentansprüche

1. Haarfärbezusammensetzung, umfassend als direkt ziehenden Farbstoff, einen direkt ziehenden Pyrazolazol-Farbstoff mit der folgenden allgemeinen Formel (1) oder ein Salz davon: 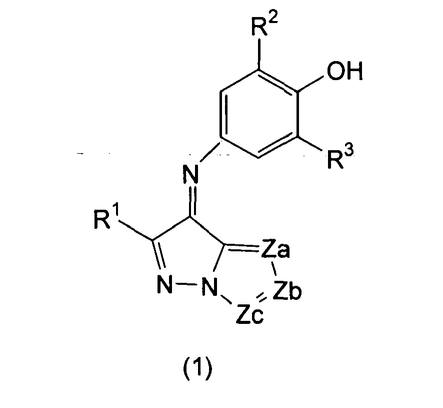 worin R¹ ein Wasserstoffatom, Halogenatom, C₁₋₅-Alkylgruppe, die wahlweise durch ein oder mehr als ein Halogenatom(e), Hydroxygruppe(n), Alkoxygruppe(n), Aryloxygruppe(n), Aminogruppe(n), Alkylaminogruppe(n), Acylgruppe(n), Acylaminogruppe(n) substituiert sein kann; C₁₋₄-Alkoxygrouppe, C₁₋₄-Alkylthiogruppe, Arylthiogruppe, Benzylthiogruppe, Acylgruppe, Acylaminogruppe, Acyloxygruppe, Carbamoylgruppe, Phenylgruppe, die wahlweise durch ein oder mehrere Halogenatom(e), Nitrogruppe(n), Sulfonylgruppe(n), C₁₋₄-Alkylgruppe(n), C₁₋₄-Alkoxygruppe(n), C₁₋₃-Trifluoralkylgruppe(n), Aminogruppe(n) oder Alkylaminogruppe(n) substituiert sein kann; Alkoxycarbonylgruppe; Aryloxycarbonylgruppe, Cyanogruppe, Nitrogruppe, Dialkylphosphinylgruppe, Alkylsulfinylgruppe, Arylsulfinylgruppe, Sulfamoylgruppe, Carboxylgruppe, Sulfogruppe, Aryloxygruppe, C₁₋₄-Alkylaminogruppe, Ureidogruppe, Sulfamoylaminogruppe, Sulfonamidogruppe, Alkoxycarbonylaminogruppe, Aryloxycarbonylaminogruppe, Heteroarylthiogruppe oder eine Phosphonylgruppe ist; worin Za, Zb and Zc jeweils unabhängig ein Stickstoffatom oder Kohlenstoffatom sind, das eine Gruppe aufweist, die oben für R¹ angegeben ist; und wobei nur eines von Zb und Zc das Kohlenstoffatom ist, wobei R² und R³ jeweils unabhängig ein Wasserstoffatom oder eine Elektronen ziehende Gruppe sind.

2. Haarfärbezusammensetzung nach Anspruch 1, worin der direkt ziehende Pyrazolazol-Farbstoff oder das Salz davon durch die folgende allgemeine Formel (2) oder (3) oder ein Salz davon definiert ist: 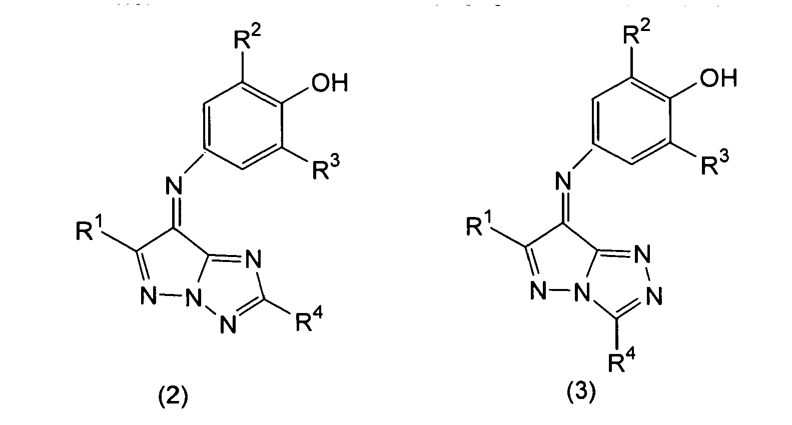 worin R¹ und R⁴ unabhängig ein Wasserstoffatom, Halogenatom, C₁₋₅-Alkylgruppe, die wahlweise durch ein oder mehrere Halogenatom(e), Hydroxygruppe(n), Alkoxygruppe(n), Aryloxygruppe(n), Aminogruppe(n), Alkylaminogruppe(n), Acylgruppe(n), Acylaminogruppe(n) substituiert sein kann; C₁₋₄-Alkoxygruppe, C₁₋₄-Alkylthiogruppe, Arylthiogruppe, Benzylthiogruppe, Acylgruppe, Acylaminogruppe, Acyloxygruppe, Carbamoylgruppe, Phenylgruppe, die wahlweise durch ein oder mehrere Halogenatom(e), Nitrogruppe(n), Sulfonylgruppe(n), C₁₋₄-Alkylgruppe(n), C₁₋₄-Alkoxygruppen, C₁₋₃-Trifluoralkylgruppe(n), Aminogruppe(n) oder Alkylaminogruppe(n) substituiert sein kann; Alkoxycarbonylgruppe, Aryloxycarbonylgruppe, Cyanogruppe, Nitrogruppe, Dialkylphosphinylgruppe, Alkylsulfinylgruppe, Arylsulfinylgruppe, Sulfamoylgruppe, Carboxylgruppe, Sulfogruppe, Aryloxygruppe, C₁₋₄-Alkylaminogruppe, Ureidogruppe, Sulfamoylaminogruppe, Sulfonamidogruppe, Alkoxycarbonylaminogruppe, Aryloxycarbonylaminogruppe, Heteroarylthiogruppe oder Phosphonylgruppe sind; wobei R² und R³ jeweils unabhängig ein Wasserstoffatom oder eine Elektronen ziehende Gruppe sind.

3. Haarfärbezusammensetzung nach Anspruch 2, worin in der allgemeinen Formel (2) oder (3) R¹ und R⁴ unabhängig ein Wasserstoffatom, eine C₁₋₅-Alkylgruppe, die wahlweise durch ein oder mehrere Hydroxygruppe(n), Alkoxygruppe(n), Aminogruppe(n) oder Alkylaminogruppe(n) substituiert sein kann; oder eine Phenylgruppe sind, die wahlweise durch ein oder mehr als zwei Halogenatom (e), C₁₋₄-Alkylgruppe (n), C₁₋₄-Alkoxygruppe (n), Aminogruppe(n) oder Alkylaminogruppe(n) substituiert sein kann; R² und R³ jeweils unabhängig ein Wasserstoffatom, Fluoratom, Chloratom, Bromatom, Jodatom, Carbonsäuregruppe oder C₁₋₄-Alkoxycarbonylgruppe sind.

4. Haarfärbezusammensetzung nach einem der Ansprüche 1-3, worin der direkt ziehende Farbstoff durch die folgenden Formeln dargestellt ist:

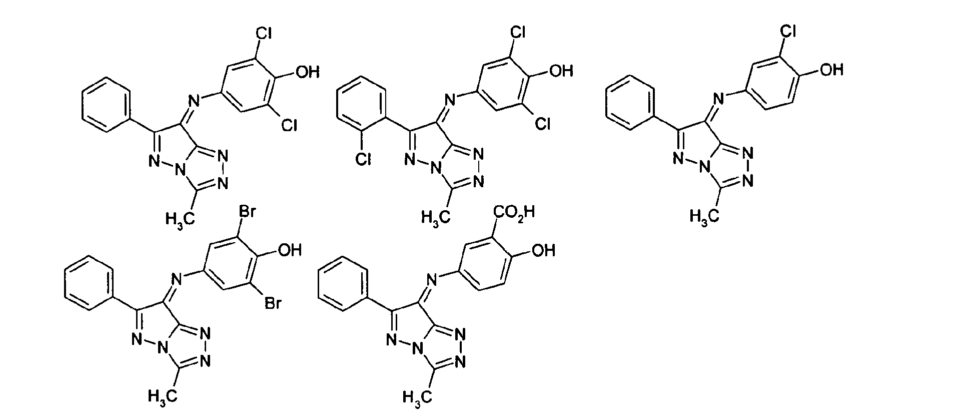

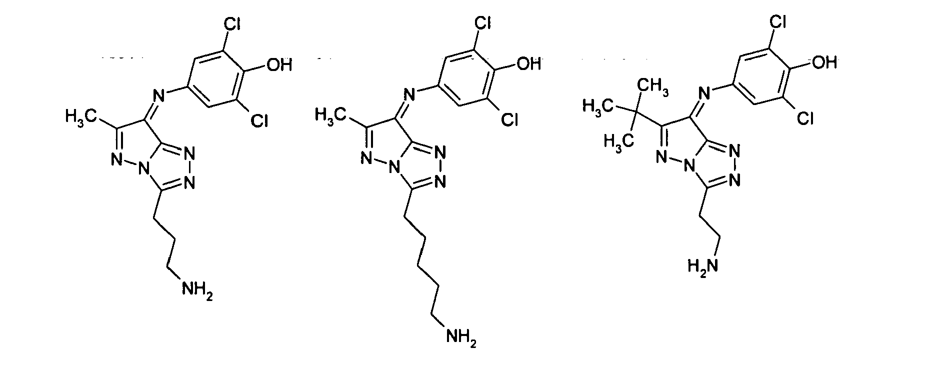

5. Haarfärbezusammensetzung nach einem der Ansprüche 1-4, umfassend 0,001 bis 5 Gew.-% von zumindest einem direkt ziehenden Pyrazolazol-Farbstoff, bezogen auf die gesamte Zusammensetzung.

6. Haarfärbezusammensetzung nach einem der Ansprüche 1-5, die weiterhin zumindest ein oxidatives Farbstoffzwischenprodukt umfasst.

7. Haarfärbezusammensetzung nach einem der Ansprüche 1-6, die weiterhin zumindest ein Peroxid aufweist und einen pH-Wert von ungefähr 7,5 bis ungefähr 12,5 aufweist.

8. Haarfärbezusammensetzung nach Anspruch 7, umfassend Wasserstoffperoxid als Peroxid.

9. Haarfärbezusammensetzung nach Anspruch 7, umfassend zumindest ein Ammoniumpersulfat, Natriumpersulfat oder Kaliumpersulfat als Peroxid.

10. Haarfärbezusammensetzung nach Anspruch 7, umfassend sowohl Wasserstoffperoxid als auch zumindest eines von Ammoniumpersulfat, Natriumpersulfat und Kaliumpersulfat als Peroxid.

11. Verfahren zum Färben von Haar mit einer Haarfärbezusammensetzung nach einem der Ansprüche 1-10.

## Revendications

1. Composition colorante pour les cheveux comprenant, comme colorant direct, un colorant direct de type pyrazoloazole représenté par la formule générale (1) ci-dessous ou un de ses sels: 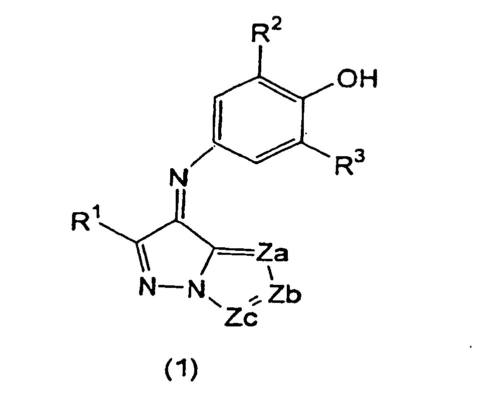 dans laquelle R¹ représente un atome d'hydrogène; un atome d'halogène; un groupe alkyle en C₁-C₅ qui peut éventuellement être substitué par un ou plusieurs atomes d'halogène, groupes hydroxy, groupes alcoxy, groupes aryloxy, groupes amino, groupes alkylamino, groupes acyle, groupes acylamino; un groupe alcoxy en C₁-C₄, un groupe alkylthio en C₁-C₄; un groupe arylthio; un groupe benzylthio; un groupe acyle; un groupe acylamino; un groupe acyloxy; un groupe carbamoyle; un groupe phényle qui peut éventuellement être substitué par un ou plusieurs atomes d'halogène, groupes nitro, groupes sulfonyle, groupes alkyle en C₁-C₄, groupes alcoxy en C₁-C₄, groupes trifluoroalkyle en C₁-C₃, groupes amino ou groupes alkylamino; un groupe alcoxycarbonyle; un groupe aryloxycarbonyle; un groupe cyano; un groupe nitro; un groupe dialkylphosphinyle; un groupe alkylsulfinyle; un groupe arylsulfinyle; un groupe sulfamoyle; un groupe carboxyle; un groupe sulfo; un groupe aryloxy; un groupe alkylamino en C₁-C₄; un groupe uréido; un groupe sulfamoylamino; un groupe sulfonamido; un groupe alcoxycarbonylamino; un groupe aryloxycarbonylamino; un groupe hétéroarylthio; ou un groupe phosphonyle; Za, Zb et Zc représentent chacun indépendamment un atome d'azote ou un atome de carbone ayant un groupe énuméré ci-dessus pour R'; et seul l'un des Zb et Zc est l'atome de carbone;
R² et R³ représentent chacun indépendamment un atome d'hydrogène ou un groupe attracteur d'électrons.

2. Composition colorante pour les cheveux selon la revendication 1, dans laquelle le colorant direct de type pyrazoloazole ou son sel est représenté par la formule générale (2) ou (3) ci-dessous ou un de ses sels: 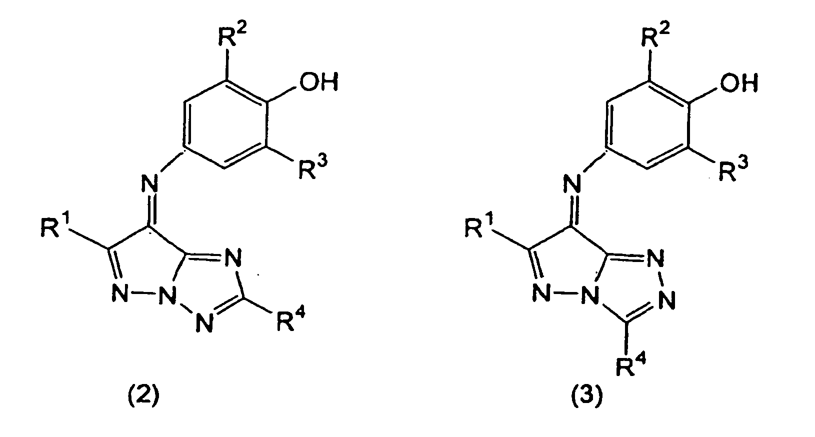 où R¹ et R⁴ représentent indépendamment un atome d'hydrogène; un atome d'halogène; un groupe alkyle en C₁-C₅ qui peut éventuellement être substitué par un ou plusieurs atomes d'halogène, groupes hydroxy, groupes alcoxy, groupes aryloxy, groupes amino, groupes alkylamino, groupes acyle, groupes acylamino; un groupe alcoxy en C₁-C₄; un groupe alkylthio en C₁-C₄; un groupe arylthio; un groupe benzylthio; un groupe acyle; un groupe acylamino; un groupe acyloxy; un groupe carbamoyle; un groupe phényle qui peut éventuellement être substitué par un ou plusieurs atomes d'halogène, groupes nitro, groupes sulfonyle, groupes alkyle en C₁₋C⁴, groupes alcoxy en C₁-C₄, groupes trifluoroalkyle en C₁-C₃, groupes amino ou groupes alkylamino; un groupe alcoxycarbonyle; un groupe aryloxycarbonyle; un groupe cyano; un groupe nitro; un groupe dialkylphosphinyle; un groupe alkylsulfinyle; un groupe arylsulfinyle; un groupe sulfamoyle; un groupe carboxyle; un groupe sulfo; un groupe aryloxy; un groupe alkylamino en C₁-C₄; un groupe uréido; un groupe sulfamoylamino; un groupe sulfonamido; un groupe alcoxycarbonylamino; un groupe aryloxycarbonylamino; un groupe hétéroarylthio; ou un groupe phosphonyle;
R² et R³ représentent chacun indépendamment un atome d'hydrogène ou un groupe attracteur d'électrons.

3. Composition colorante pour les cheveux selon la revendication 2 dans laquelle, dans la formule générale (2) ou (3), R¹ et R⁴ représentent indépendamment un atome d'hydrogène; un groupe alkyle en C₁-C₅ qui peut éventuellement être substitué par un ou plusieurs groupes hydroxy, groupes alcoxy, groupes amino ou groupes alkylamino; ou un groupe phényle qui peut éventuellement être substitué par un ou plus de deux atomes d'halogène, groupes alkyle en C₁-C₄, groupes alcoxy en C₁-C₄, groupes amino ou groupes alkylamino; R² et R³ représentent indépendamment un atome d'hydrogène, un atome de fluor, un atome de chlore, un atome de brome, un atome d'iode, un groupe acide carboxylique ou un groupe (alcoxy en C₁-C₄)carbonyle.

4. Composition colorante pour les cheveux selon l'une quelconque des revendications 1 à 3, dans laquelle le colorant direct est représenté par les formules suivantes:

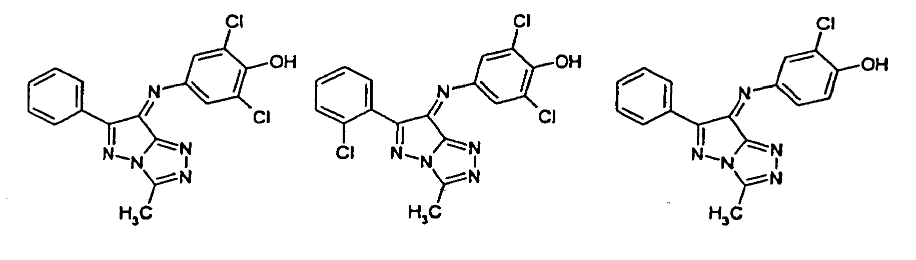

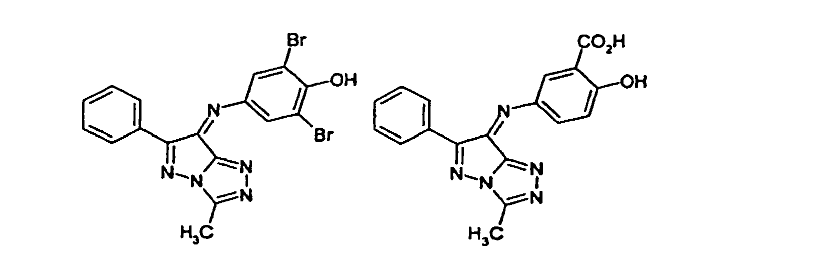

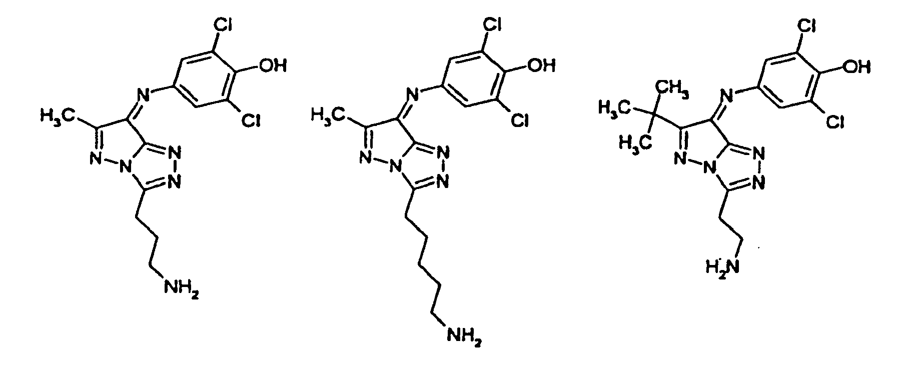

5. Composition colorante pour les cheveux selon l'une quelconque des revendications 1 à 4, qui comprend 0,00 1 à 5 % en poids d'au moins un colorant direct de type pyrazoloazole par rapport à la composition totale.

6. Composition colorante pour les cheveux selon l'une quelconque des revendications 1 à 5, qui comprend en outre au moins un intermédiaire de colorant oxydant.

7. Composition colorante pour les cheveux selon l'une quelconque des revendications 1 à 6, qui comprend en outre au moins un peroxyde et a une valeur de pH d'environ 7,5 à environ 12,5.

8. Composition colorante pour les cheveux selon la revendication 7, qui comprend du peroxyde d'hydrogène comme peroxyde.

9. Composition colorante pour les cheveux 7, qui comprend comme peroxyde au moins un parmi persulfate d'ammonium, persulfate de sodium et persulfate de potassium.

10. Composition colorante pour les cheveux 7, qui comprend comme peroxyde à la fois du peroxyde d'hydrogène et au moins un parmi persulfate d'ammonium, persulfate de sodium et persulfate de potassium.

11. Procédé de coloration des cheveux avec une composition colorante pour les cheveux selon l'une quelconque des revendications 1 à 10.
